(19) Europäisches Patentamt
European Patent Office
Office européen des brevets

(11) Publication number: **0 400 978 A2**

(12) # EUROPEAN PATENT APPLICATION

(21) Application number: 90305861.8

(51) Int. Cl.[5]: C07H 17/08, A01N 43/90

(22) Date of filing: 30.05.90

(30) Priority: 02.06.89 US 360624

(43) Date of publication of application: 05.12.90 Bulletin 90/49

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Blizzard, Timothy A.
2126 Allen Street
Rahway, NJ 07065(US)

(74) Representative: Hesketh, Alan, Dr. et al
European Patent Department Merck & Co., Inc. Terlings Park Eastwick Road
Harlow Essex, CM20 2QR(GB)

(54) **Avermectin -3,4-oxide derivatives useful as antiparasitic agents and insecticides.**

(57) There are disclosed avermectin derivatives in which the 3,4-double bond of the avermectins has been epoxidized. The compounds are prepared by oxidation of 7-O-trimethylsilyl-avermectins followed by deprotection. The compounds are active antiparasitic agents and insecticides and compositions for these uses are disclosed.

EP 0 400 978 A2

Xerox Copy Centre

# AVERMECTIN-3,4-OXIDE DERIVATIVES USEFUL AS ANTIPARASITIC AGENTS AND INSECTICIDES

## BACKGROUND OF THE INVENTION

The avermectins (previously referred to as C-076 compounds) are a series of compounds produced by fermentation of avermectin producing strains of Streptomyces avermitilis and derivatives thereof. The morphological characteristics of the culture are completely described in U.S. Pat. No. 4,310,519. The production, isolation, and structure determination of the avermectins are fully described in Albers-Schonberg et al. J. Am. Chem. Soc. 103, 4216-4221 (1981) and references cited therein. The conversion of natural avermectin B1 to 22,23-dihydro-avermectin B1, the potent broad spectrum anthelminthic agent known as ivermectin, has also been described in the literature (Chabala et al J. Med. Chem. 23, 1134-1136 1980). The naturally occurring avermectins and the instant derivatives thereof have a very high degree of anthelminthic and anti-parasitic activity.

The naturally occurring avermectins are a series of macrocyclic lactones which are substituted at position 13 with a disaccharide consisting of two oleandrose residues. The preparation and properties of synthetic avermectin aglycones in which the disaccharide moiety has been removed leaving a free hydroxl group at position 13 have been described by Mrozik et al J. Org. Chem. 47, 489-492 (1982) and by Chabala et al J. Med. Chem. 23, 1134-1136 (1982). Furthermore, the preparation and properties of synthetic avermectin analogs in which the 8,9 double bond and/or the 14,15 double bond have been epoxidized were described by Mrozik U.S. Patent 4,530,921. The natural compounds have the following general structure:

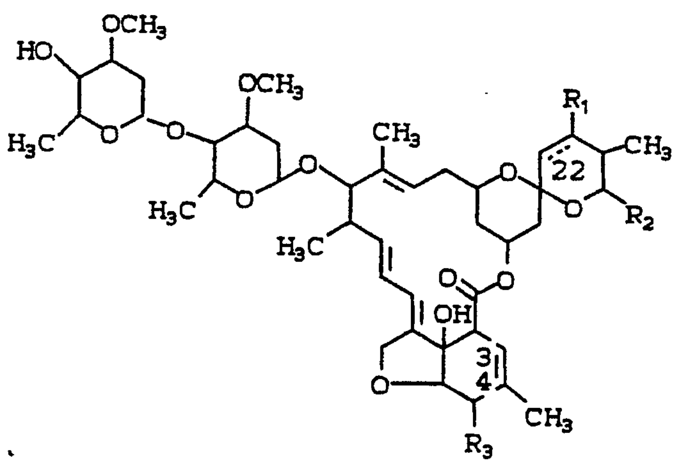

wherein the broken line indicates a single or double bond;
$R_1$ is hydroxy and is present only when said broken line indicates a single bond;
$R_2$ is iso-propyl or sec-butyl; and
$R_3$ is methoxy or hydroxy.

There are eight major natural avermectin compounds, designated A1a, A1b, A2a, A2b, B1a, B1b, B2a and B2b. These designations are based on the structure of the individual compounds as shown in the following table (referring to the foregoing structural formula).

| Compound | broken line | $R_1$ | $R_2$ | $R_3$ |
|---|---|---|---|---|
| A1a | double bond | ---- | sec-butyl | $--OCH_3$ |
| A1b | double bond | ---- | iso-propyl | $--OCH_3$ |
| A2a | single bond | OH | sec-butyl | $--OCH_3$ |
| A2b | single bond | OH | iso-propyl | $--OCH_3$ |
| B1a | double bond | ---- | sec-butyl | --OH |
| B1b | double bond | ---- | iso-propyl | --OH |
| B2a | single bond | OH | sec-butyl | --OH |
| B2b | single bond | OH | iso-propyl | --OH |

The avermectins are generally isolated as mixtures of the a and b components (typically ≧80% a and ≦20% b). Such compounds differ only in the nature of the $R_2$ substituent and this minor structural difference has been found to have very little effect on the chemical reactivity or biological activity of the compounds. Thus although the a and b components can be separated from each other by chromatography this is not necessary and hence is not normally done. The presence of a mixture of a and b components is indicated by dropping the a or b from the designation of the compound. A mixture of avermectin B1a and avermectin B1b is thus referred to as avermectin B1.

A related family of natural products is known as the milbemycins. The milbemycins have the same basic cyclic structure as the avermectins but have no substitution at position 13 and have a methyl or ethyl group at position 25 (R2 = methyl or ethyl rather than isopropyl or sec-butyl as in the avermectins). The milbemycins and the fermentation conditions used to prepare them are described in U.S. Pat. No. 3.950,360. Closely related 13-deoxy-avermectin aglycones are prepared by chemical modification of the natural avermectins and have been described in U.S. Pat. Nos. 4,171,134 and 4,173,571.

Recently a number of related compounds have been described in European Patent Application EPO 170,006 and U.K. aplication 2,166,436 (see also Carter et al, J. Antibiotics 1988, 41, 519-529). These compounds are essentially 13-deoxy-avermectin aglycones in which the $R_2$ side chain contains a double bond and, in some cases, includes additional carbon atoms.

## SUMMARY OF THE INVENTION

This invention is concerned with certain avermectin derivatives in which the double bond between carbons 3 and 4 has been epoxidized and the use of these derivatives as antiparasitic agents and insecticides. Thus it is an object of this invention to describe these avermectin derivatives. A further object of this invention is to describe processes for the preparation of these compounds. A still further object is to describe the use of the instant compounds as antiparasitic agents in the treatment and prevention of parasitic diseases. A still further object is to describe compositions for the treatment of parasitic diseases which contain the novel compounds of this invention as the active ingredient thereof. A still further object is to describe the use of the instant compounds as insecticides. Further objects will become apparent from a reading of the following description.

## DESCRIPTION OF THE INVENTION

The compounds of the instant invention are best realized in the following structure:

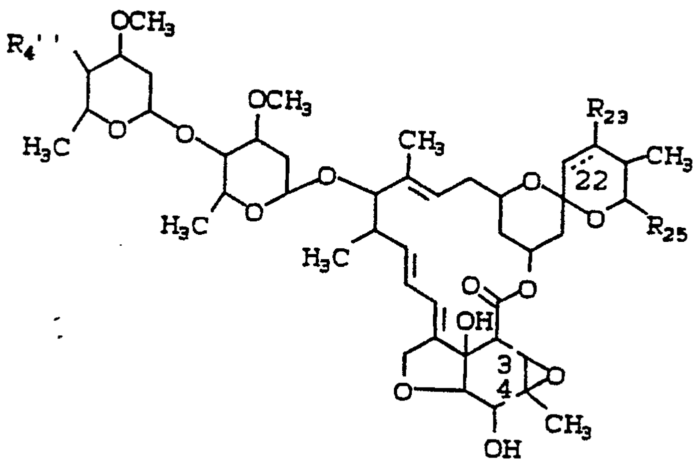

wherein the broken line indicates a single or double bond and;
$R_4''$ = OH, $NH_2$, NH-loweralkyl, NH-loweralkanoyl;
$R_{23}$ = H, OH and $R_{23}$ is present only if the broken line indicates a single bond;
$R_{25}$ = loweralkyl

In the instant invention "lower alkyl" is intended to include those alkyl groups of from 1 to 7 carbon atoms in either a straight or branched chain. Examples of such alkyl groups are methyl, ethyl, propyl, isopropyl, butyl, sec-butyl, pentyl, hexyl, heptyl, and the like.

The term "lower alkanoyl" is intended to include those alkanoyl groups of from 1 to 7 carbon atoms in either a straight or branched chain. Examples of such alkanoyl groups are formyl, acetyl, propionyl, isopropionyl, butyrl, sec-butyrl, pentanoyl, hexanoyl, heptanoyl, and the like.

Preferred compounds of this invention are realized when:
$R_4''$ = OH, $NH_2$, NH-lower alkyl, NH-lower alkanoyl;
$R_{23}$ = H or OH; (provided that $R_{23}$ is present only when the broken line indicates a single bond;
$R_{25}$ = loweralkyl;
and the broken line indicates a single or double bond

More preferred compounds of this invention are realized when:
$R_4''$ = OH, NH-loweralkyl, NH-loweralkanoyl;
$R_{23}$ = H;
$R_{25}$ = isopropyl, sec-butyl;
and the broken line indicates a single or double bond

Still more preferred compounds of this invention are realized when:
$R_4''$ = OH, $NHCH_3$, NH-acetyl;
$R_{23}$ = H;
and the broken line indicates a single or double bond.

The most preferred compounds of this invention are realized when:
$R_4''$ = OH
and the broken line indicates a single or double bond

Examples of the preferred compounds of this invention are as follows:
avermectin B1 3,4-oxide
22,23-dihydro-avermectin B1 3,4-oxide
avermectin B2 3,4-oxide
4″-amino-avermectin B1 3,4-oxide
4″-methylamino-avermectin B1 3,4-oxide
4″-acetylamino-avermectin B1 3,4-oxide
4″-acetylamino-avermectin B2 3,4-oxide

## PREPARATION OF STARTING MATERIALS

The starting materials for this invention are disclosed in Albers-Schonberg et al J. Am. Chem. Soc. 103, 4216-4221 (1981) and references cited therein (naturally occurring avermectins), and Chabala et al J. Med. Chem. 23, 1134-1136 (1980) (22,23-dihydro-avermectin B1 (ivermectin).

The novel compounds of this invention are prepared by the following procedures:

Selective epoxidation of the C-3,4 double bond is effected by a directed epoxidation reaction utilizing the hydroxyl group at postion 5 as the directing group. It is therefore necessary to protect the hydroxyl group at position 7 in order to prevent preferential epoxidation at the C-8,9 olefin. The protecting group must be stable to the oxidation conditions yet readily removed after the reaction is complete. Triloweralklysilyl protecting groups are acceptable, trimethylsilyl is preferred. The necessary 7-O-trimethylsilyl derivatives are prepared by treating the avermectin with a large excess of a strong silylating agent such as bis-trimethylsilyl-trifluoroacetamide (BSTFA), bis-trimethylsilyl-acetamide (BSA), and the like in a dipolar aprotic solvent such as dimethylformamide, dimethylsulfoxide, and the like at temperatures ranging from 20° C to 40° C for 10 to 60 hours. The solvent is then removed under high vacuum leaving a residue which consists of a per-silylated derivative (all hydroxyl groups converted to trimethylsilyl ethers). The resulting per-trimethylsilyl ether is then dissolved in an ethereal solvent such as tetrahydrofuran, ether, and the like to which a small amount of water (1-15%) has been added and treated with an acid such as p-toluenesulfonic acid, hydrochloric acid, and the like for 2 to 45 minutes at temperatures ranging from 0° C to 30° C in order to convert secondary silyl ethers to the free hydroxyl group while leaving the tertiary silyl ether at position 7 intact. The reaction is then worked up and the product purified using standard techniques known to those skilled in the art.

With the C-7 hydroxyl protected as a triloweralkylsilyl ether the hydroxyl group at C-5 can be used, with the appropriate catalyst, to direct epoxidation to the C-3,4 olefin. The directing catalyst is a transition metal cation, such as vanadium, titanium, and the like, which coordinates to the C-5 hydroxyl group and to the oxidant and delivers the oxidant, an alkyl hydroperoxide and the like, to the C-3,4 olefin. Preferred catalysts are vanadium complexes such as vanadyl acetylacetonate and vanadium acetylacetonate. The preferred oxidant is tert-butyl hydroperoxide. The 7-O-trimethylsilyl avermectin derivative is dissolved in a halogenated solvent such as dichloromethane, chloroform, and the like. The vanadium catalyst (from 0.1 to 1 molar equivalents) is then added followed by from 1 to 5 equivalents of the oxidizing agent. The mixture is stirred at temperatures ranging from 20° C to 40° C for 10 to 60 hours. In some cases it is necessary to add additional catalyst and/or oxidizing agent in order to drive the reaction to completion. The reaction is then worked up and the product purified using standard techniques known to those skilled in the art.

After the oxidation is complete and the epoxide isolated and purified it is necessary to regenerate the C-7 hydroxyl group by removing the trimethylsilyl protecting group. This is accomplished by treating a solution of the silyl ether in an ethereal solvent such as tetrahydrofuran, ethyl ether, and the like with a fluoride source such as hydrogen fluoride, potassium fluoride, tetrabutylammonium fluoride, and the like with or without the addition of a base such as triethyl amine, pyridine and the like for 1 to 48 hours at temperatures ranging from 10° C to 35° C. Alternatively, the protecting group may be removed by treatment with a solution of p-toluenesulfonic acid (0.5-2%) in methanol at 0° C to 25° C for 0.5 to 8 hours. Deprotection with hydrogen fluoride in tetrahydrofuran with added pyridine is preferred.

An amino substituent may be introduced at position 4″ by reductive amination of a 4″-ketone which is in turn prepared by oxidation of the 4″-hydroxyl group present in the avermectins. During the oxidation of the hydroxyl group at C-4″ it is necessary to protect other secondary hydroxyl groups in the molecule (note that it is not necessary to protect the tertiary hydroxyl present at position 7) with a protecting group which may be removed after the oxidation is accomplished. Suitable protecting groups include tert-butyldimethylsilyl, tert-butyldiphenylsilyl, phenoxyacetyl, acetyl, and the like. The tert-butyldimethylsilyl group is preferred and is introduced by treating a solution of the alcohol in dimethylformamide (DMF) with an excess of imidazole and a silylating reagent such as tertbutyldimethylsilyl-chloride, tert-butyl-dimethylsilyl-trifluoromethanesulfonate, and the like at temperatures ranging from 25° C to 50° C for 4 to 48 hours. The reaction is then worked up and the product isolated and purified using standard techniques known to those skilled in the art. The protecting group may be removed by treatment with a solution of hydrogen fluoride in a pyridine/tetrahydrofuran solvent mixture. Alternatively, the protecting group may be removed by treatment with a solution of p-toluenesulfonic acid (0.5-2%) in methanol at 0° C to 25° C for 0.5 to 8 hours. Deprotection with hydrogen fluoride in pyridine/tetrahydrofuran is preferred. In both cases reaction workup and product isolation and purification are by standard techniques well known to those skilled in the art.

With other secondary hydroxyl groups protected as a silyl ether the hydroxyl group at position 4″ can be oxidized by a variety of methods to afford the ketone derivatives necessary for conversion to amino and acylamino analogs. The oxidation of this hydroxyl group can be effected by using a variety of oxidation procedures, including oxidation with dimethylsulfoxide (DMSO) based systems commonly known to those skilled in the art as Swern (or Moffat) oxidations (DMSO-oxalyl-chloride, DMSO-acetic anhydride, DMSO-trifluoroacetic anhydride and the like) as well as oxidations with chromium based reagents (pyridinium chlorochromate, pyridinium dichromate, and the like), or other methods known to those skilled in the art.

The DMSO based oxidations are preferred. The oxidation reagent is generated by treating a solution of DMSO in a non-nucleophilic solvent such as dichloromethane, chlorform, ether (preferred), tetrahydrofuran and the like with an electrophilic activating agent such as oxalyl chloride (preferred), dicyclohexylcarbodiimide (DCC), phosgene, and the like at temperatures ranging from -90° C to -55° C and stirring the mixture thus formed at this temperature for 10 to 90 minutes. To the oxidizing reagent thus generated is added, at the same temperature, a solution of the alcohol in the solvent used to generate the reagent. The solution is stirred at temperatures ranging form -90° C to -55° C for 10 to 90 minutes then a hindered base such as triethylamine, diisopropylethylamine, and the like is added. The temperature is raised to 0° C to 30° C and the mixture stirred at this temperature for 10 to 90 minutes. The reaction is then worked up and the product isolated and purified using standard techniques known to those skilled in the art.

The 4″-ketone functionality thus generated may be used to introduce amino substituents at position 4″ via a reductive amination reaction. The reductive amination is accomplished by treating a solution of the ketone in an alcoholic solvent such as methanol, ethanol, and the like with an ammonium salt such as ammonium acetate (preferred), ammonium formate, ammonium benzoate and the like at temperatures ranging from -25° C to 25° C for 15 to 60 minutes then adding sodium cyanoborohydride to the resulting mixture and stirring at temperatures ranging from 0° C to 30° C for 30 to 90 minutes. The reaction is then worked up and the product isolated and purified using standard techniques known to those skilled in the art. The reaction may be modified by substituting an alkylammonium salt in the place of ammonium acetate in the above procedure to prepare avermectin derivatives substituted with an alkylamino group at the 4″ position.

The amino (or alkylamino) substituted derivatives prepared as described above may be acylated to provide acylamino analogs. The acylation is accomplished by treating a solution of the 4″-amino or 4″-alkylamino analog in a halogenated solvent such as dichloromethane, chloroform or the like with one molar equivalent of an acylating agent such as an alkanoyl chloride (preferred), alkanoyl bromide, alkanoic acid in combination with dicyclohexylcarbodiimide, and the like in the presence of a base such as triethylamine, pyridine and the like with or without the addition of a nucleophilic catalyst such as dimethylaminopyridine at temperatures ranging from -10° C to 35° C for 15 minutes to 24 hours. The reaction is then worked up and the product isolated and purified using standard techniques known to those skilled in the art. Note that it is not necessary to protect secondary alcohols in the molecule during the acylation reaction as the amino functionality is sufficiently more reactive that acylation occurs selectively at nitrogen.

The instant compounds of this invention are unexpectedly potent antiparastic agents against endo and ecto parasites, particularly helminths and arthropods, which cause numerous parasitic diseases in humans, animals, and plants.

Parasitic diseases may be caused by either endoparasites or ectoparasites. Endoparasites are those parasites which live inside the body of the host, either within an organ (such as the stomach, lungs, heart, intestines, etc.) or simply under the skin. Ectoparasites are those parasites which live on the outer surface of the host but still draw nutrients from the host.

The endoparasitic diseases generally referred to as helminthiasis are due to infection of the host with parasitic worms known as helminths. Helminthiasis is a prevalent and serious worldwide economic problem due to infection of domesticated animals such as swine, sheep, horses, cattle, goats, dogs, cats, and poultry. Many of these infections are caused by the group of worms described as nematodes which cause diseases in various species of animals throughout the world. These diseases are frequently serious and can result in the death of the infected animal. The most common genera of nematodes infecting the animals referred to above are Haemonchus, Trichostrongylus,Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris, and Parascaris. Many parasites are species specific (infect only one host) and most also have a preferred site of infection within the animal. Thus Haemonchus and Ostertagia primarily infect the stomach while Nematodirus and Cooperia mostly attack the intestines. Other parasites prefer to reside in the heart, eyes, lungs, blood vessels, and the like while still others are subcutaneous parasites. Helminthiasis can lead to weakness, weight loss, anemia, intestinal damage, malnutrition, and damage to other organs. If left untreated these diseases can result in the death of the animal.

Infections by ectoparasitic arthropods such as ticks, mites, lice, stable flies, hornflies, blowflies, fleas, and the like are also a serious problem. Infection by these parasites results in loss of blood, skin lesions, and can interfere with normal eating habits thus causing weight loss. These infections can also result in transmission of serious diseases such as encephalitis, anaplasmosis, swine pox, and the like which can be fatal.

Animals may be infected by several species of parasite at the same time since infection by one

parasite may weaken the animal and make it more susceptible to infection by a second species of parasite. Thus a compound with a broad spectrum of activity is particularly advantageous in the treatment of these diseases. The compounds of this invention have unexpectedly high activity against these parasites, and in addition are also active against Dirofilaria in dogs, Nematospiroides and Syphacia in rodents, biting insects, and migrating diperous larvae such as Hypoderma sp. in cattle, and Gastrophilus in horses.

The instant compounds are also useful against endo and ecto parasites which cause parasitic diseases in humans. Examples of such endoparasites which infect man include gastrointestinal parasites of the genera Ancylostoma, Necator, Ascaris, Strongyloides, Trichinella, Capillaria, Trichuris, Enterobius, and the like. Other endoparasites which infect man are found in the blood or in other organs. Examples of such parasites are the filarial worms Wucheria, Brugia, Onchocerca, and the like as well as extra-intestinal stages of the intestinal worms Strongylides and Trichinella. Ectoparasites which parasitize man include arthropods such as ticks, fleas, mites, lice, and the like and, as with domestic animals, infections by these parasites can result in transmission of serious and even fatal diseases. The instant compounds are active against these endo and ecto parasites and in addition are also active against biting insects and other dipterous pests which annoy humans.

The instant compounds are also useful against common household pests such as Blatella sp. - (cockroach), Tineola sp. (clothes moth), Attagenus sp.(carpet beetle), Musca domestica (housefly) and against Solenopsis Invicta (imported fire ant).

The compounds are furthermore useful against agricultural pests such as aphids (Acyrthiosiphon sp.), locusts, and boll weevils as well as against insect pests which attack stored grains such as Tribolium sp. and against immature stages of insects living on plant tissue. The compounds are also useful as a nematodicide for the control of soil nematodes which may be agriculturally important.

For use as an antiparasitic agent in animals the instant compounds may be administered internally either orally or by injection, or topically as a liquid pour-on or as a shampoo.

For oral administration, the compounds may be administered in capsule, tablet, or bolus form or alternatively they can be mixed in the animals feed. The capsules, tablets, and boluses are comprised of the active ingredient in combination with an appropriate carrier vehicle such as starch, talc, magnesium stearate, or di-calcium phosphate. These unit dosage forms are prepared by intimately mixing the active ingredient with suitable finely-powdered inert ingredients including diluents, fillers, disintegrating agents, and/or binders such that a uniform mixture is obtained. An inert ingredient is one that will not react with the instant compounds and which is non-toxic to the animal being treated. Suitable inert ingredients include starch, lactose, talc, magnesium stearate, vegetable gums and oils, and the like. These formulations may contain a widely variable amount of the active and inactive ingredients depending on numerous factors such as the size and type of the animal species to be treated and the type and severity of the infection. The active ingredient may also be administered as an additive to the feed by simply mixing the compound with the feedstuff or by applying the compound to the surface of the feed. Alternatively the active ingredient may be mixed with an inert carrier and the resulting composition may then either be mixed with the feed or fed directly to the animal. Suitable inert carriers include corn meal, citrus meal, fermentation residues, soya grits, dried grains and the like. The active ingredients are intimately mixed with these inert carriers by grinding, stirring, milling, or tumbling such that the final composition contains from 0.0001 to 5% by weight of the active ingredient.

The compounds may alternatively be administered parenterally via injection of a formulation consisting of the active ingredient dissolved in an inert liquid carrier. Injection may be either intramuscular, intraruminal, intratracheal, or subcutaneous. The injectable formulation consists of the active ingredient mixed with an appropriate inert liquid carrier. Acceptable liquid carriers include the vegetable oils such as peanut oil, cotton seed oil, sesame oil and the like as well as organic solvents such as solketal, glycerol formal and the like. As an alternative, aqueous parenteral formulations may also be used. The vegetable oils are the preferred liquid carriers. The formulations are prepared by dissolving or suspending the active ingredient in the liquid carrier such that the final formulation contains from 0.001 to 10% by weight of the active ingredient.

Topical application of the instant compounds is possible through the use of a liquid drench or a shampoo containing the instant compounds as an aqueous solution or suspension. These formulations generally contain a suspending agent such as bentonite and normally will also contain an antifoaming agent. Formulations containing from 0.005 to 10% by weight of the active ingredient are acceptable. Preferred formulations are those containing from 0.01 to 5% by weight of the instant compounds.

The instant compounds are primarily useful as antiparasitic agents for the treatment and/or prevention of helminthiasis in domestic animals such as cattle, sheep, horses, dogs, cats, goats, swine, and poultry. They are also useful in the prevention and treatment of parasitic infections of these animals by ectoparasites such as ticks, mites, lice, fleas and the like. They are also effective in the treatment of parasitic infections of humans. In treating such infections the compounds of this invention may be used individually or in combination with each other or with other unrelated antiparasitic agents. The dosage of the instant compounds required for best results depends on several factors such as the species and size of the animal, the type and severity of the infection, the method of administration and the compound used. Oral administration of the instant compounds at a dose level of from 0.0005 to 10 mg per kg of animal body weight, either in a single dose or in several doses spaced a few days apart, generally gives good results. A single dose of one of the instant compounds normally gives excellent control however repeat doses may be given to combat re-infection or for parasite species which are unusually persistent. The techniques for administering these compounds to animals are known to those skilled in the veterinary field.

The compounds of this invention may also be used to combat agricultural pests which attack crops either in the field or in storage. The compounds are applied for such uses as sprays, dusts, emulsions and the like either to the growing plants or the harvested crops. The techniques for applying these compounds in this manner are known to those skilled in the agricultural arts.

The following examples are provided in order that this invention might be more fully understood; they are not to be construed as limitative of the invention. The avermectin derivatives prepared in the following examples are generally isolated as amorphous solids rather than crystalline solids. They are characterized analytically using techniques such as nuclear magnetic resonance, mass spectrometry, and the like. Being amorphous the compounds are not characterized by sharp melting points but the chromatographic and analytical methods employed indicate that they are pure.

## EXAMPLE 1

### 7-O-Trimethylsilyl-avermectin B1:

Bis(trimethylsilyl)trifluoromethylacetamide (4.56 ml, BSTFA) was added to a solution of avermectin B1 (1.0 g) in 10 ml of dry dimethylformamide (DMF) and the resulting solution stirred at room temperature for 6 hours. The solvent and excess reagent were then evaporated under high vacuum. The resulting yellow oil was dissolved in 15 ml of a solvent mixture consisting of 90% tetrahydrofuran and 10% water then 58 mg of p-toluenesulfonic acid was added. The solution was stirred at room temperature for 20 minutes (analytical thin layer chromatography indicated that the reaction was complete after 10 minutes) then partitioned between ether (35 ml) and 5% aqueous $NaHCO_3$ (10 ml). The layers were separated and the aqueous layer extracted with ether (3 x 20 ml). The combined extracts were dried with $MgSO_4$, filtered and evaporated under vacuum. The residue was purified by flash column chromatography on silica gel eluted with 33% acetone in hexane to afford 1.05 g (97% yield) of a white foam which was identified by $^1H$ NMR and MS as 7-O-trimethylsilyl-avermectin B1. Elemental analysis: calculated for $C_{51}H_{80}O_{14}Si$: C, 64.80; H, 8.53. Found: C, 64.55; H, 8.82.

## EXAMPLE 2

### 7-O-Trimethylsilyl-avermectin B1 3,4-oxide

Vanadyl acetylacetonate (295 mg) was added to a solution of 7-O-trimethylsilyl-avermectin B1 (1.05 g) in 25 ml of dichloromethane then tert-butyl hydroperoxide (1.11 ml of a 3 M solution in toluene) was added and the solution stirred at room temperature. Analytical thin layer chromatography after 21 hours at room temperature showed that the reaction was not complete so additional tert-butyl hydroperoxide (1.11 ml of a 3 M solution in toluene) was added and the solution stirred at room temperature for an additional 3.5 hours. The mixture was diluted with dichloromethane (40 ml) then 5% aqueous $NaHCO_3$ was added. The layers were separated and the aqueous layer extracted with dichloromethane (2 x 20 ml). The combined extracts were dried with $MgSO_4$, filtered and evaporated under vacuum. The yellow oily residue was purified by flash column chromatography on silica gel eluted with 33% acetone in hexane. Evaporation of the fractions containing pure product afforded 0.15 g (14% yield) of a white foam which was identified by $^1H$ NMR and

MS as pure 7-O-trimethylsilyl-avermectin B1 3,4-oxide. Evaporation of fractions containing impure product afforded an additional 0.15 g of less pure material. Elemental analysis: calculated for $C_{51}H_{80}O_{15}Si$: C, 63.72; H, 8.39. Found: C, 63.71; H, 8.58.

EXAMPLE 3

Avermectin B1 3.4-oxide:

A solution of 7-O-trimethylsilyl-avermectin B1 3,4-oxide (150 mg) in 0.5 ml of a solvent mixture consisting of 10% hydrogen fluoride•pyridine complex, 20% pyridine, and 70% tetrahydrofuran was stirred at room temperature for two hours. The mixture was diluted with ether (2 ml) then 5% aqueous $NaHCO_3$ was added. The layers were separated and the aqueous layer extracted with ether (2 ml). The combined extracts were dried with $MgSO_4$, filtered and evaporated under vacuum. The residue was purified by preparative thin layer chromatography on a 1.0 mm silica gel plate eluted with 40% acetone in hexane. The product band was isolated to afford 46 mg of a white foam which was identified by $^1H$ NMR and MS as avermectin B1 3, 4-oxide. Elemental analysis: calculated for $C_{48}H_{72}O_{15}$: C, 64.85; H, 8.16. Found: C, 64.54; H, 8.44.

EXAMPLE 4

7-O-Trimethylsilyl-22,23-dihydro-avermectin B1:

Substitution of 22,23-dihydro-avermectin B1 (ivermectin) for avermectin B1 in the silylation procedure of Example 1 affords 7-O-trimethylsilyl-22,23-dihydro-avermectin B1 which is identified by $^1H$ NMR and MS.

EXAMPLE 5

7-O-Trimethylsilyl-22,23-dihydro-avermectin B1 3,4-oxide

Substitution of 7-O-trimethylsilyl-22,23-dihydro-avermectin B1 for 7-O-trimethylsilyl-avermectin B1 in the epoxidation procedure of Example 2 affords 7-O-trimethylsilyl-22,23-dihydro-avermectin B1 3,4-oxide which is identified by $^1H$ NMR and MS.

EXAMPLE 6

22,23-Dihydro-avermectin B1 3,4-oxide:

Substitution of 7-O-trimethylsilyl-22,23-dihydro-avermectin B1 3,4-oxide for 7-O-trimethylsilyl-avermectin B1 3,4-oxide in the deprotection procedure of Example 3 affords 22,23-dihydro-avermectin $B_1$ 3,4-oxide which is identified by $^1H$ NMR and MS.

EXAMPLE 7

7-O-Trimethylsilyl-avermectin B2:

Substitution of avermectin B2 for avermectin B1 in the silylation procedure of Example 1 affords 7-O-trimethylsilyl-avermectin B2 which is identified by $^1$H NMR and MS.

EXAMPLE 8

7-O-Trimethylsilyl-avermectin B2 3,4-oxide:

Substitution of 7-O-trimethylsilyl-avermectin B2 for 7-O-trimethylsilyl-avermectin B1 in the epoxidation procedure of Example 2 affords 7-O-trimethylsilyl-avermectin B2 3,4-oxide which is identified by $^1$H NMR and MS.

EXAMPLE 9

Avermectin B2 3,4-oxide:

Substitution of 7-O-trimethylsilyl-avermectin B2 3,4-oxide for 7-O-trimethylsilyl-avermectin B1 3,4-oxide in the deprotection procedure of example 3 affords avermectin B2 3,4-oxide which is identified by $^1$H NMR and MS.

EXAMPLE 10

5-O-t-Butyldimethylsilyl-avermectin B1-3,4-oxide:

tert-Butyldimethylsilyl chloride (54 mg) is added to a solution of avermectin B1 3,4-oxide (250 mg) and imidazole (48 mg) in 5 ml of dry dimethylformamide and the solution is stirred at room temperature for 24 hours. The reaction mixture is partitioned between ether (50 ml) and water (50 ml). The aqueous layer is extracted with ether (30 ml) and the combined organic layers dried with magnesium sulfate, filtered and evaporated. The crude product is purified by preparative layer chromatography on two 2.0 mm silica gel plates eluted with 25% acetone in hexane to afford 5-O-t-butyldimethylsilyl-avermectin B1 3,4-oxide, which is identified by $^1$H NMR and mass spectrometry.

EXAMPLE 11

4″-Oxo-4″-deoxy-avermectin B1-3,4-oxide:

A solution of dimethylsulfoxide (0.053 ml) in 1 ml of dichloromethane is added to a cold (-78°C) solution of oxalyl chloride (0.031 ml) in 2 ml of dichloromethane. The solution is stirred at -78°C for 20 minutes then a solution of 5-O-t-butyldimethylsilyl-avermectin B1 3,4-oxide (225 mg) in 3 ml of dichloromethane is added. The solution is stirred at -78°C for 45 minutes then triethylamine (0.225 ml) is added and the cold bath is removed. The solution is stirred at room temperature for 45 minutes then poured into water (20 ml) and extracted with dichloromethane (3 x 10 ml). The combined extracts are dried with magnesium sulfate, filtered and evaporated. The crude oxidation product is dissolved in 2.0 ml of tetrahydrofuran then 2.0 ml of a solvent mixture consisting of 10% hydrogen fluoride•pyridine complex, 20% pyridine, and 70% tetrahydrofuran is added. The mixture is stirred at room temperature for 24 hours.

The mixture is diluted with ether (5 ml) then 5% aqueous $NaHCO_3$ (5 ml) is added. The layers are separated and the aqueous layer is extracted with ether (2 x 4 ml). The combined extracts are dried with $MgSO_4$, filtered and evaporated under vacuum to afford 4″-oxo-4″-deoxy-avermectin B1 3,4-oxide, which is identified by $^1H$ NMR and mass spectrometry. This material is used without further purification in the reductive amination procedure of example 12.

## EXAMPLE 12

### 4″-Amino-4″-deoxy-avermectin B1-3,4-oxide:

Ammonium acetate (175 mg) and 3A molecular sieves are added to a solution of the crude 4″-oxo-4″-deoxy-avermectin B1 3,4-oxide from example 11 in 3 ml of methanol. The mixture is stirred at room temperature for 30 minutes then sodium cyanoborohydride (45 mg) is added in 3 small portions at intervals of 5 minutes. The solution is stirred at room temperature for two hours then centrifuged. The supernatant is decanted off and partitioned between dichloromethane (10 ml) and water (5 ml). The layers are separated and the aqueous layer is extracted with dichloromethane (2 x 4 ml). The combined extracts are dried with $MgSO_4$, filtered and evaporated under vacuum. The crude product is purified by silica gel chromatography to afford 4″-amino-4″deoxy-avermectin B1 3,4-oxide, which is identified by $^1H$ NMR and mass spectrometry.

## EXAMPLE 13

### 4″-Methylamino-4″-deoxy-avermectin B1-3,4-oxide:

Substitution of methylammonium acetate for ammonium acetate in the reductive amination procedure of example 12 affords 4″-methylamino-4″-deoxy-avermectin B1 3,4-oxide, which is identified by $^1H$ NMR and mass spectrometry.

## EXAMPLE 14

### 4″-Acetylamino-4″-deoxy-avermectin B1-3,4-oxide:

Acetyl chloride (0.008 ml) is added to a cold (0 °C) solution of 4″-amino-4″-deoxy-avermectin B1 3,4-oxide (100 mg) and triethylamine (0.031 ml) in 3 ml of dichloromethane. The solution is stirred at 0 °C for 1 hour then water (3 ml) is added. The layers are separated and the aqueous layer is extracted with dichloromethane (2 x 3 ml). The combined extracts are dried with $MgSO_4$, filtered and evaporated under vacuum. The crude product is purified by preparative layer silica gel chromatography to afford 4″-acetylamino-4″-deoxy-avermectin B1 3,4-oxide, which is identified by $^1H$ NMR and mass spectrometry.

## Claims

1. A compound having the formula:

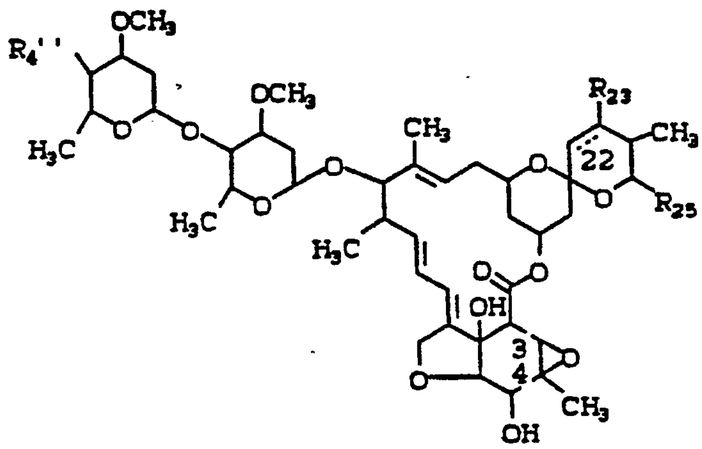

wherein:
$R_4''$ = OH, $NH_2$, NH-lower alkyl, NH-lower alkanoyl;
$R_{23}$ = H or OH and $R_{23}$ is present only when the broken line indicates a single bond;
$R_{25}$ = lower alkyl;
and the broken line indicates a single or double bond

2. The compound of Claim 1 wherein
$R_4''$ = OH, NH-lower alkyl, NH-lower alkanoyl;
$R_{23}$ = H;
$R_{25}$ = isopropyl, sec-butyl;
and the broken line indicates a single or double bond

3. The compound of Claim 2 wherein
$R_4''$ = OH, $NHCH_3$, NH-acetyl;
$R_{23}$ = H;
and the broken line indicates a single or double bond

4. The compound of Claim 3 wherein $R_4''$ = OH, and the broken line indicates a single or io double bond

5. The compound of Claim 1 which is avermectin B1 3,4-oxide

6. The use of a compound of Claim 1 for the preparation of a medicament useful for the treatment and/or prevention of parasitic infections in animals.

7. A method for the treatment of pests of plants which comprises treating said plants or the soil in which they grow with an effective amount of a compound of Claim 1.

8. A composition useful for the treatment and/or prevention of parasitic infections of animals which is comprised of an inert carrier and a compound of Claim 1.

9. A composition useful for the treatment of pests of plants which is comprised of an inert carrier and a compound of Claim 1.

10. A process for the preparation of a compound of Claim 1 comprises the selective epoxidation of the corresponding compound containing a 3,4-double bond.